# EUROPEAN PATENT APPLICATION

(11) **EP 2 524 645 A1**
(43) Date of publication of application: **21.11.2012**
(21) Application number: 12168488.0
(22) Date of filing: 18.05.2012
(51) Int. Cl.: A61B 1/005, G02B 23/24, A61B 1/273, A61B 1/31, A61B 1/00, A61B 1/04

(54) **Flexible-tubed structure of endoscope**

(30) Priority: 18.05.2011 TW 100117310; 18.05.2011 TW 100117311
(71) Applicant: Three-In-One Enterprises Co., Ltd., New Taipei City 241 (TW)
(72) Inventor: Chen, Yung-Hsiu, 241 New Taipei City (TW); Weng, Kuo-Chen, 241 New Taipei City (TW)
(74) Representative: Schwerbrock, Florian

(57) **Abstract**

The present invention relates to a flexible-tubed structure of an endoscope comprising a plurality of cylindrical units (11), a first wire (31), a second wire (32), a first flexible unit (21), and a second flexible unit (22). The wires (31, 32) respectively pass through the cylindrical units (11). The flexible units (21, 22) are continuously disposed at the recesses (112, 113, 114, 115) of the cylindrical units (11). The cylindrical units (11) are used to form a tubular unit (1) by the flexible units (21, 22). The tubular unit (1) is bent by the tension of the first wire (31) or the second wire (32) to control the turning-direction (61, 62, 63, 64) of the camera apparatus (4).

## Description

### FIELD OF THE INVENTION

This invention relates to a structure of an endoscope, and in particular, to a flexible-tubed structure of an endoscope.

### BACKGROUND OF THE INVENTION

The invention of the endoscope makes humans being available to observe the inner side of objects clearly. In clinical, the clinicians can make diagnosis by using the images from endoscope, such as the inspections of esophagus endoscope, stomach endoscope, and colon endoscope. In industrial applications, users can use the endoscope to watch the inner side of engines or somewhere which is hardly observed with naked eyes. Owing to see multi-direction images clearly, the endoscope nowadays often has the function of turning direction.

However, nowadays flexible structures are mostly made with the combination of ring metals, rivets, or springs. Take the Taiwan patent M352352 for example. It provides a turning-direction endoscope device comprising with a turning-direction component, a elastic component, a supporting part, a first wire, a second wire, and a dual-direction flexible component. By using the combination of the components, the endoscope can have the function of turning directions. However, there are still some problems which are described below.

Using the springs as the elastic component. The springs will break due to the elastic fatigue after frequent use, thereby affecting the operation of the turning-direction device.

It is necessary to add a component of dual-direction flexible component in previous inventions to make the endoscope turning direction. It will make the production process complicated and have high costs.

This invention provides a flexible-tubed structure of an endoscope to improve the traditional problems.

### SUMMARY OF THE INVENTION

The invention provides a flexible-tubed structure of an endoscope. The flexible-tubed structure of an endoscope comprises a plurality of cylindrical units and a plurality of flexible units to form a tubular unit. The flexible units are used to keep the tubular unit straight. The flexible-tubed structure of an endoscope also comprises a plurality of wires to control a turning-direction of a camera apparatus.

The flexible-tubed structure of an endoscope comprises a plurality of cylindrical units, a first wire, a second wire, a first flexible unit, and a second flexible unit.

The each cylindrical unit includes a central hole in the center and a first recess and a second recess respectively disposed at the outside of the each cylindrical unit. The first wire and the second wire respectively pass through the cylindrical units. The first flexible unit is continuously disposed at the first recesses of the cylindrical units. The second flexible unit is continuously disposed at the second recesses of the cylindrical units. Wherein, the cylindrical units are used to form a tubular unit kept straight by the first flexible unit and the second flexible unit, and a distance is between the every two cylindrical units.

In an embodiment, the each cylindrical unit comprises a first hole and a second hole near the central hole. The first wire passes through the first holes of the cylindrical units, and the second wire passes through the second holes of the cylindrical units.

In an embodiment, the flexible-tubed structure further comprises a camera apparatus including an endo-lens and a control module. The control module is used to process the image signal captured from the endo-lens, and the image signal is transmitted by a signal wire. One end of the first wire and the second wire are connected to the camera apparatus, the tubular unit is bent by the tension of the first wire or the second wire to control the turning-direction of the camera apparatus.

In an embodiment, the first wire or the second wire can be steel wire.

In an embodiment, the first flexible unit or the second flexible unit is flat-shaped, the material of the first flexible unit or the second flexible unit is shape memory alloy (SMS) or galvanized iron. The first flexible unit and the second flexible unit are welded to continuously embed in the first recesses and the second recesses of the cylindrical units.

The advantages of the flexible-tubed structure of an endoscope in the invention are as follows:

The structure of the endoscope is only composed of a plurality of cylindrical units and flexible units which is simpler than the conventional technology. It can effectively decrease the cost and manufacturing processes.

The flat-shaped flexible units of the invention are continuously disposed at the first recesses of the cylindrical units to form a tubular unit. The bent tubular unit can recover to straight status by the flexible units.

Since the distance is between every two cylindrical units, and tubular unit is formed by the plurality of cylindrical units, the tubular unit is bent by the tension of the first wire or the second wire to control the turning-direction of the camera apparatus. It can achieve the same effect as the conventional technology using spring, but the using life and the stability of the invention are larger than the using life and the stability of the conventional technology.

These and other features, aspects and advantages of the disclosure will become better understood with regard to the following description, appended claims, and accompanying drawings.

### BRIEF DESCRIPTION OF THE DRAWINGS

Fig. 1 is a schematic showing a first embodiment of the flexible-tubed structure of an endoscope in the invention.

Fig. 2 shows a cross-sectional view of the cylindrical unit in the invention.

Fig. 3 is a diagram showing the flexible-tubed structure of an endoscope with the camera apparatus in the invention.

Fig. 4 shows a cross-sectional view showing a second embodiment of the cylindrical unit 11 in the invention.

Fig. 5 is a diagram showing a second embodiment of the tubular unit in the invention.

Fig. 6 shows a cross-sectional view showing a second embodiment of the tubular unit in the invention.

Fig.7 is a diagram showing a second embodiment of the flexible-tubed structure of an endoscope with the camera apparatus in the invention.

### DETAILED DESCRIPTION OF THE INVENTION

For clarity of disclosure, and not by way of limitation, the detailed description of the invention is divided into the subsections that follow.

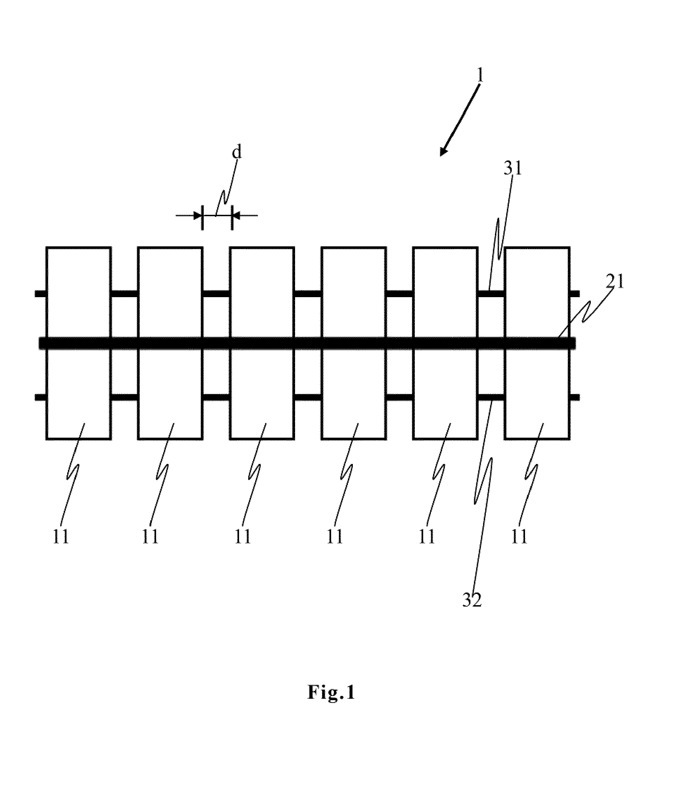

Fig.1 is a diagram showing a first embodiment of the flexible-tubed structure of an endoscope in the invention. The flexible-tubed structure of an endoscope comprises a plurality of cylindrical units 11, a first wire 31 and a second wire 32, a first flexible unit 21, a second flexible unit (not shown). The cylindrical units 11 are used to form a tubular unit 1 kept straight by the first flexible unit 21 and the second flexible unit, and a distance d is between the every two cylindrical units 11.

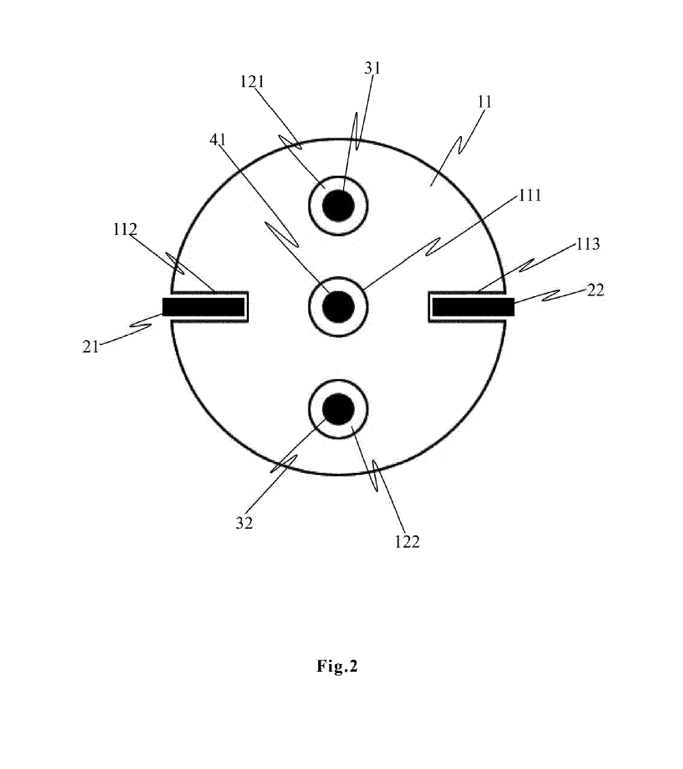

Fig.2 is a cross-sectional view of the cylindrical unit 11 in the invention. The each cylindrical unit 11 includes a central hole 111 in the center for a signal wire 41 passing through. The each cylindrical unit 11 comprises a first hole 121 and a second hole 122 near the central hole 111. In an embodiment, the first hole 121 is disposed above the central hole 111, and the second hole 122 is disposed below the central hole 111. The first hole 121, the second hole 122 and the central hole 111 are arranged in a straight line, but not limited to in the invention.

The first holes 121 are used to be passed through by the first wire 31, and the second holes 122 are used to be passed through by the second wire 32. The first wire 31 or the second wire 32 can be steel wire, but not limited to in the invention.

A first recess 112 and a second recess 113 are respectively disposed at the outside of the each cylindrical unit 11. The first flexible unit 21 is embedded in the first recesses 112 and the second flexible unit 22 is embedded in the second recesses 113. The indented shape of the recess is corresponding to the shape of the flexible unit. For example, if the shape of the flexible unit is circular, the indented shape of the recess is also circular. If the shape of the flexible unit is square, the indented shape of the recess is also square, but not limited to in the invention.

In an embodiment, the first flexible unit 21 and the second flexible unit 22 are flat-shaped, the material of the first flexible unit 21 and the second flexible unit 22 are shape memory alloy (SMS) or galvanized iron. Therefore, the tubular unit 1 is formed by keeping a distance d between the every two cylindrical units 11, the first flexible unit 21 being continuously disposed at the first recesses 112 of the cylindrical units 11, the second flexible unit 22 being continuously disposed at the second recesses 113 of the cylindrical units 11, then by welding or sticking the above elements to fix and embed with each other stably.

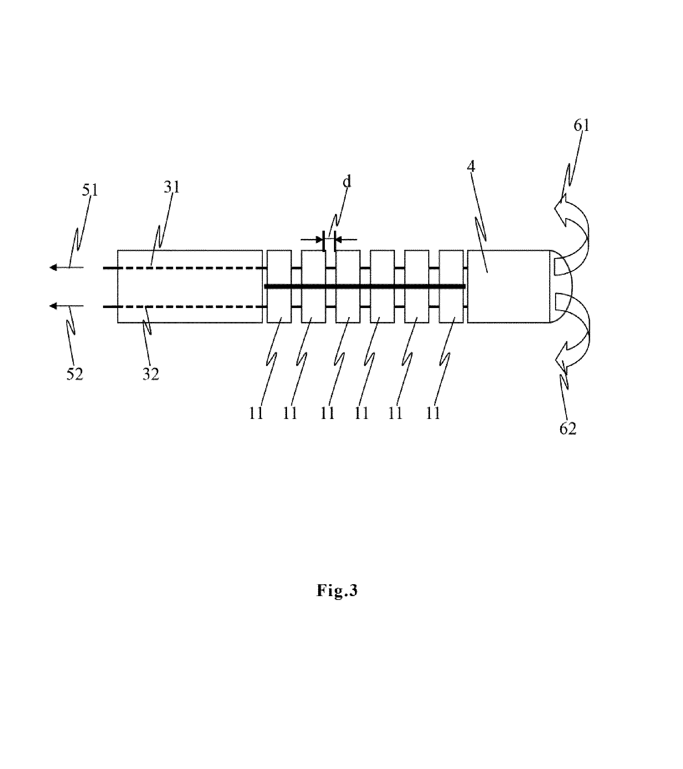

The flexible-tubed structure further comprises a camera apparatus 4. Please refer to Fig.3, and the Fig.3 is a diagram showing the flexible-tubed structure of an endoscope with the camera apparatus in the invention. The camera apparatus 4 includes an endo-lens and a control module. The control module is used to process the image signal captured from the endo-lens, and the image signal is transmitted by a signal wire 41. The signal wire 41 passes through the central holes 111 of the cylindrical units 11 and further transmits the image signal to display on a mobile apparatus.

One end of the first wire 31 and the second wire 32 are connected to the camera apparatus 4. The first wire 31 passes through the first holes 121 of the cylindrical units 11, and the second wire 32 passes through the second holes 122 of the cylindrical units 11. Therefore, the tubular unit 1 is bent by the tension of the first wire 31 and the second wire 32 to control the turning-direction of the camera apparatus 4.

As shown in Fig. 3, there is a distance d between every two cylindrical units 11. When the first wire 31 provides a force toward to a first wire direction 51, since the distance d between every two cylindrical units 11, there is space for bending. As a result, the camera apparatus 4 can be bent toward to a first turning-direction 61. Similarly the second wire 32 can provide a force toward to a second wire direction 52, and the camera apparatus 4 can be bent toward to a second turning-direction 62. Therefore, the camera apparatus 4 can be bent toward to several directions, and can achieve the same effect as the conventional technology using spring.

The bent tubular unit 1 can recover to straight status by flexible units. Moreover because the distance d between every two cylindrical units 11, if the first flexible unit 21 or the second flexible unit 22 breaks or cracks, the first wire 31 or the second wire 32 can be used to support the tubular unit 1, and not to affect the bending function. The using life and the stability of the invention are larger than the using life and the stability of the conventional technology.

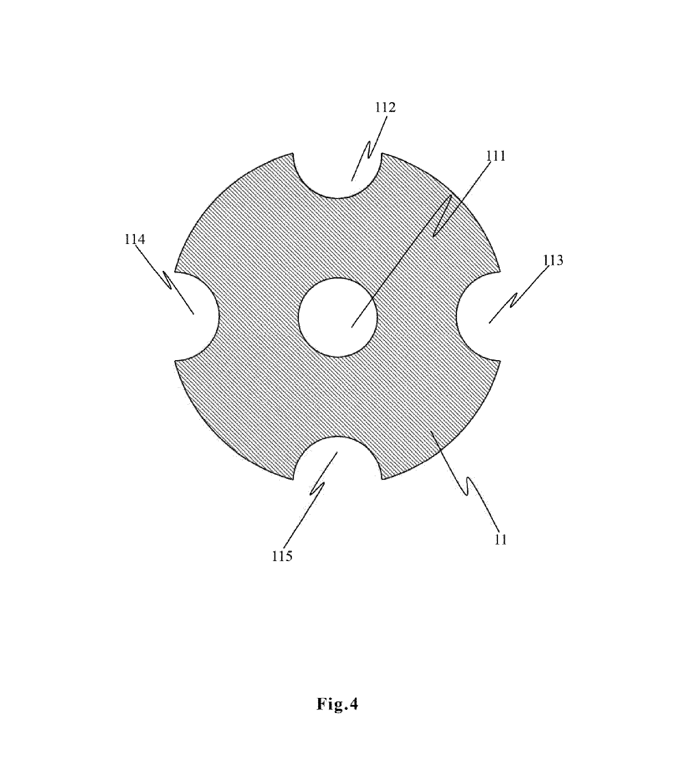

Please refer to Fig. 4, and the Fig. 4 is a cross-sectional view showing a second embodiment of the cylindrical unit 11 in the invention. The each cylindrical unit 11 includes a central hole 111 in the center. A first recess 112, a second recess 113, a third recess 114 and a fourth recess 115 are respectively disposed at the outside of the each cylindrical unit 11.

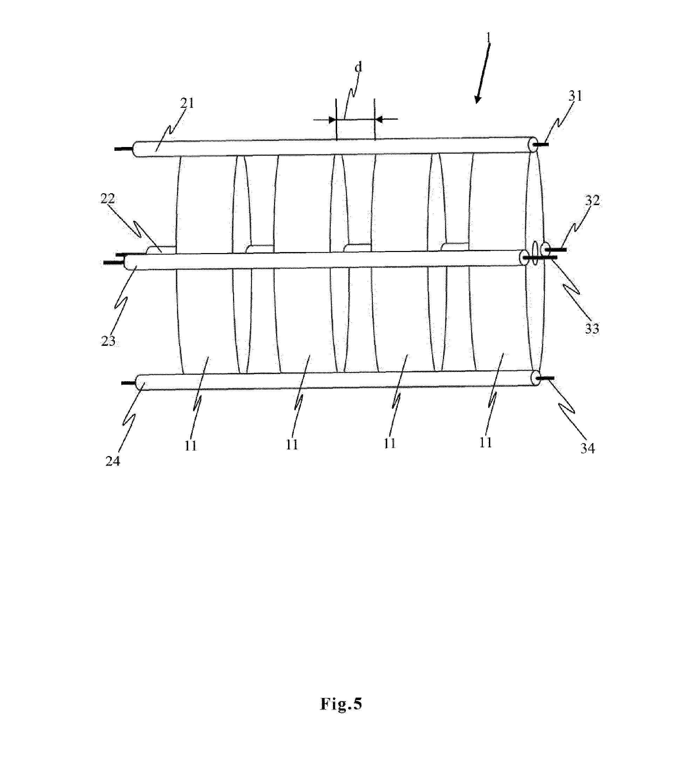

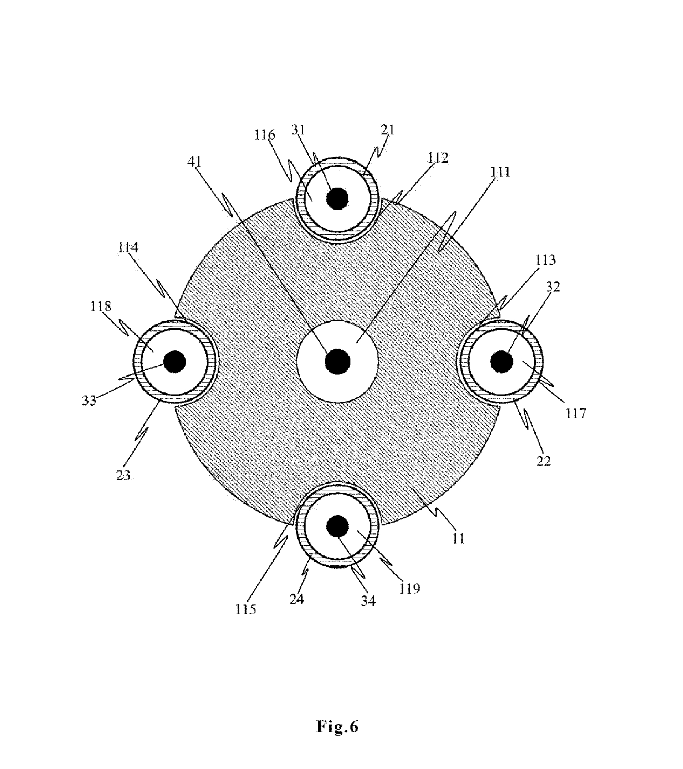

Please refer to Fig. 5 and Fig. 6. The Fig. 5 is a diagram showing a second embodiment of the tubular unit in the invention, and the Fig. 6 is a cross-sectional view showing a second embodiment of the tubular unit in the invention. The tubular unit 1 is formed by keeping a distance d between the every two cylindrical units 11, a first flexible unit 21, a second flexible unit 22, a third flexible unit 23, and a fourth flexible unit 24 being continuously disposed at first recesses 112, second recesses 113, third recesses 114, and fourth recesses 115 of the cylindrical units 11.

The indented shape of the recess is corresponding to the shape of the flexible unit. For example, if the shape of the flexible unit is circular, the indented shape of the recess is also circular. If the shape of the flexible unit is square, the indented shape of the recess is also square, but not limited to in the invention.

In the embodiment, the indented shape of the recess is circular, and the shape of the flexible unit is also circular. The flexible units are welded to continuously embed in the recesses of the cylindrical units, so the he flexible units can keep the tubular unit 1 straight.

The first flexible unit 21 includes a first container 116 for the first wire 31 passing through. The second flexible unit 22 includes a second container 117 for the second wire 32 passing through. The third flexible unit 23 includes a third container 118 for the third wire 33 passing through. The fourth flexible unit 24 includes a fourth container 119 for the fourth wire 34 passing through. The first wire 31, the second wire 32, the third wire 33 or the fourth wire 34 can be steel wire. The first flexible unit 21, the second flexible unit 22, the third flexible unit 23 or the fourth flexible unit 24 can be spring.

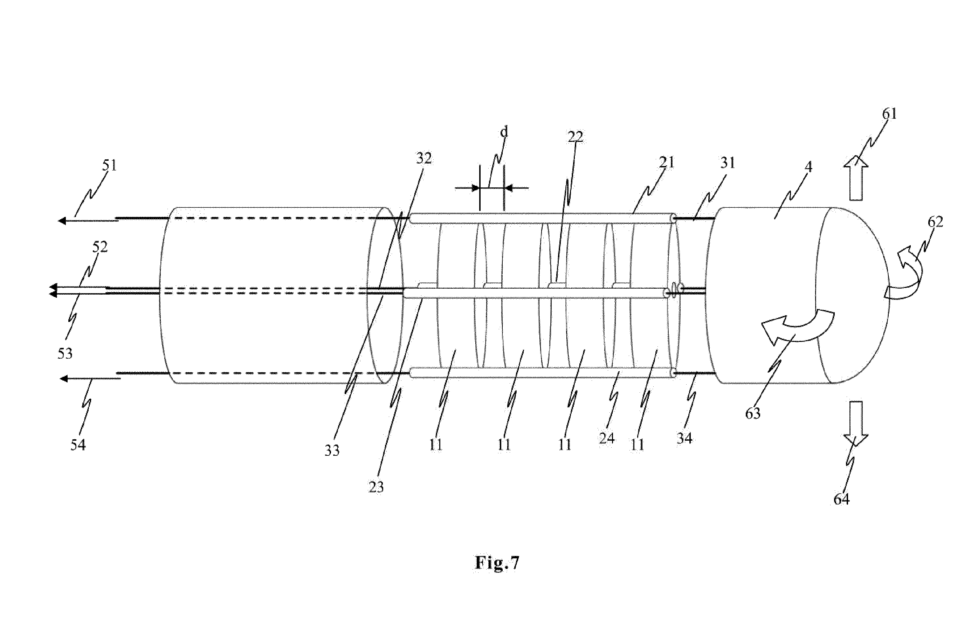

Fig.7 is a diagram showing a second embodiment of the flexible-tubed structure of an endoscope with the camera apparatus in the invention. The flexible-tubed structure further comprises a camera apparatus 4. The camera apparatus 4 includes an endo-lens and a control module. The control module is used to process the image signal captured from the endo-lens, and the image signal is transmitted by a signal wire 41. The signal wire 41 passes through the central holes 111 of the cylindrical units 11.

One end of the first wire 31, the second wire 32, the third wire 33 and the fourth wire 34 are respectively connected to the camera apparatus 4 and respectively pass through the first container 116, the second container 117, the third container 118 and the fourth container 119. The tubular unit 1 is bent by the tension of the first wire 31, the second wire 32, the third wire 33 and the fourth wire 34 to control the turning-direction of the camera apparatus 4.

As shown in Fig. 7, there is a distance d between every two cylindrical units 11. When the first wire 31 provides a force toward to a first wire direction 51, since the distance d between every two cylindrical units 11, there is space for bending. As a result, the camera apparatus 4 can be bent toward to a first turning-direction 61. Similarly the second wire 32, the third wire 33, and the fourth wire 34 can respectively provide a force toward to a second wire direction 52, a third wire direction 53, and a fourth wire direction 54, and the camera apparatus 4 can be respectively bent toward to a second turning-direction 62, a third turning-direction 63 and a fourth turning-direction 64.

Therefore, the camera apparatus 4 can be bent toward to several turning-directions, can achieve the same effect as the conventional technology using spring, and can avoid the situation of the conventional technology that when the only one spring breaks, the operation of turning direction also breaks.

The flexible-tubed structure of an endoscope in the invention includes many flexible units, and wires passed through the flexible units. Even the flexible unit breaks or cracks, the wire can be used to support the tubular unit 1, and not to affect the bending function. The using life and the stability of the invention are larger than the using life and the stability of the conventional technology. The structure of the endoscope is simpler than the conventional technology. It can effectively decrease the cost and manufacturing processes.

Although the disclosure has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the disclosure.

## Claims

1. A flexible-tubed structure of an endoscope, comprising:
a plurality of cylindrical units, the each cylindrical unit having a central hole in the center and a first recess and a second recess respectively disposed at the outside of the each cylindrical unit;
a first wire and a second wire respectively passed through the cylindrical units;
a first flexible unit continuously disposed at the first recesses of the cylindrical units; and
a second flexible unit continuously disposed at the second recesses of the cylindrical units;
wherein, the cylindrical units are used to form a tubular unit kept straight by the first flexible unit and the second flexible unit, and a distance is between the every two cylindrical units.

2. The flexible-tubed structure according to claim 1, wherein the each cylindrical unit comprises a first hole and a second hole near the central hole, the first wire and the second wire respectively pass through the first holes and the second holes of the cylindrical units.

3. The flexible-tubed structure according to claim 2, further comprises a camera apparatus including an endo-lens and a control module, the control module is used to process the image signal captured from the endo-lens, and the image signal is transmitted by a signal wire.

4. The flexible-tubed structure according to claim 3, wherein one end of the first wire and the second wire are connected to the camera apparatus, the tubular unit is bent by the tension of the first wire or the second wire to control the turning-direction of the camera apparatus.

5. The flexible-tubed structure according to claim 3, wherein the signal wire passes through the central holes of the cylindrical units.

6. The flexible-tubed structure according to claim 2, wherein the first wire or the second wire is steel wire.

7. The flexible-tubed structure according to claim 2, wherein the first flexible unit or the second flexible unit is spring.

8. The flexible-tubed structure according to claim 2, wherein the first flexible unit is flat-shaped, the material of the first flexible unit is shape memory alloy (SMS) or galvanized iron.

9. The flexible-tubed structure according to claim 2, wherein the second flexible unit is flat-shaped, the material of the second flexible unit is shape memory alloy (SMS) or galvanized iron.

10. The flexible-tubed structure according to claim 1, wherein the first flexible unit and the second flexible unit are welded to continuously embed in the first recesses and the second recesses of the cylindrical units.

11. The flexible-tubed structure according to claim 1, further comprises:
a third recess and a fourth recess are respectively disposed at the outside of the each cylindrical unit;
a third flexible unit and a fourth flexible unit, the first flexible unit, the second flexible unit, the third flexible unit and the fourth flexible unit are continuously disposed at the first recesses, the second recesses, the third recesses and the fourth recesses of the cylindrical units respectively, and have a first container, a second container, a third container and a fourth container respectively; and
a third wire and a fourth wire, the first wire, the second wire, the third wire and the fourth wire respectively pass through the first container, the second container, the third container and the fourth container;
wherein, the cylindrical units are used to form the tubular unit kept straight by the first flexible unit, the second flexible unit, the third flexible unit and the
fourth flexible unit, and the distance is between the every two cylindrical units.

12. The flexible-tubed structure according to claim 11, further comprises a camera apparatus including an endo-lens and a control module, the control module is used to process the image signal captured from the endo-lens, and the image signal is transmitted by a signal wire.

13. The flexible-tubed structure according to claim 12, wherein one end of the first wire, the second wire, the third wire and the fourth wire are connected to the camera apparatus, the tubular unit is bent by the tension of the first wire, the second wire, the third wire and the fourth wire to control the turning-direction of the camera apparatus.

14. The flexible-tubed structure according to claim 12, wherein the signal wire passes through the central holes of the cylindrical units.

15. The flexible-tubed structure according to claim 11, wherein the first wire, the second wire, the third wire or the fourth wire is steel wire.

16. The flexible-tubed structure according to claim 11, wherein the first flexible unit, the second flexible unit, the third flexible unit or the fourth flexible unit is spring.

17. The flexible-tubed structure according to claim 11, wherein the first flexible unit, the second flexible unit, the third flexible unit and the fourth flexible unit are welded to continuously embed in the first recesses, the second recesses, the third recesses and the fourth recesses of the cylindrical units.
